(19) Europäisches Patentamt
European Patent Office
Office européen des brevets

(11)  **EP 3 037 550 B1**

(12)  **EUROPEAN PATENT SPECIFICATION**

(45) Date of publication and mention
of the grant of the patent:
**27.12.2017  Bulletin 2017/52**

(51) Int Cl.:
*C12Q 1/68* (2006.01)

(21) Application number: **15167379.5**

(22) Date of filing: **28.07.2010**

(54)  **DETERMINATION OF THE RISK OF DISTANT METASTASES IN SURGICALLY TREATED PATIENTS WITH NON-SMALL CELL LUNG CANCER IN STAGE I-IIIA**

**BESTIMMUNG DES RISIKOS ENTFERNTER METASTASEN BEI CHIRURGISCH BEHANDELTEN PATIENTEN MIT NICHT-KLEINZELLIGEM LUNGENKREBS DES STADIUMS I-IIIA**

**DÉTERMINATION DU RISQUE DE MÉTASTASES DISTANTES CHEZ DES PATIENTS TRAITÉS CHIRURGICALEMENT ET AYANT UN CANCER DU POUMON NON À CELLULES PETITES AU STADE I-IIIA**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **30.07.2009  PL 38868109**

(43) Date of publication of application:
**29.06.2016  Bulletin 2016/26**

(62) Document number(s) of the earlier application(s) in accordance with Art. 76 EPC:
**10752447.2 / 2 459 748**

(73) Proprietors:
• **Skrzypski, Marcin**
81721 Sopot (PL)
• **Jassem, Jacek**
80-157 Gdansk (PL)
• **Gdanski Uniwersytet Medyczny**
80-210 Gdansk (PL)

(72) Inventors:
• **Skrzypski, Marcin**
81-721 Sopot (PL)
• **Jassem, Jacek**
80-157 Gdansk (PL)

(56) References cited:
**WO-A2-2007/081720**

• **YANAIHARA NOZOMU ET AL: "Unique microRNA molecular profiles in lung cancer diagnosis and prognosis", CANCER CELL, CELL PRESS, US, vol. 9, no. 3, 1 March 2006 (2006-03-01), pages 189-198, XP002510059, ISSN: 1535-6108, DOI: DOI:10.1016/J.CCR2006.01.025**
• **MARKOU ATHINA ET AL: "Prognostic value of mature microRNA-21 and microRNA-205 overexpression in non-small cell lung cancer by quantitative real-time RT-PCR", CLINICAL CHEMISTRY, vol. 54, no. 10, October 2008 (2008-10), pages 1696-1704, XP002611193, ISSN: 0009-9147**
• **TAKAMIZAWA J ET AL: "Reduced expression of the let-7 microRNAs in human lung cancers in association with shortened postoperative survival", CANCER RESEARCH, AMERICAN ASSOCIATION FOR CANCER RERERACH, US, vol. 64, 1 June 2004 (2004-06-01), pages 3753-3756, XP002385720, ISSN: 0008-5472, DOI: DOI:10.1158/0008-5472.CAN-04-0637**
• **YU SUNG-LIANG ET AL: "MicroRNA signature predicts survival and relapse in lung cancer", CANCER CELL JAN 2008 LNKD- PUBMED:18167339,, vol. 13, no. 1, 1 January 2008 (2008-01-01), pages 48-57, XP002608382,**
• **MA LI ET AL: "Tumour invasion and metastasis initiated by microRNA 10b in breast cancer", NATURE, NATURE PUBLISHING GROUP, LONDON, GB, vol. 449, no. 7163, 11 October 2007 (2007-10-11), page 682, XP002547671, ISSN: 0028-0836, DOI: DOI:10.1038/NATURE06174 [retrieved on 2007-09-26]**

EP 3 037 550 B1

• **BAFFA RAFFAELE ET AL: "MicroRNA expression profiling of human metastatic cancers identifies cancer gene targets", 1 June 2009 (2009-06-01), JOURNAL OF PATHOLOGY, JOHN WILEY & SONS LTD, GB, PAGE(S) 214 - 221, XP002598261, ISSN: 0022-3417 [retrieved on 2009-06-01] * abstract; table 2 ***

**Description**

**FIELD OF THE INVENTION AND THE ESSENCE**

The present invention is within the general area of medical genetics and in the fields of biotechnology and oncology. More specifically, it relates to the method of determination of the risk of distant metastases and determination of prognosis in surgically treated patients with non-small cell lung cancer (NSCLC) in stage I-IIIA. The risk of relapse and prognosis are established by relating the expression of the microRNAs in the sample of primary tumor tissue to the reference expression values of the listed microRNAs (in a model of distant recurrence prediction). In this model, expressions of particular microRNAs are correlated with either the high or low risk of distant metastases and thus with patient's prognosis.

Background

Lung cancer is the most common cause of cancer-related mortality in both women and men. Yearly, there are 20,000 new cases in Poland, 80% of whom are diagnosed with NSCLC. In Poland and in several other European countries, squamous cell lung cancer is the most prevalent histological type among surgically treated patients.

In early stages, surgery (lobectomy or pneumonectomy) is the treatment of choice, however even in this group the treatment results are unsatisfactory, with approximately only 50% of patients surviving 5 years.

The most common pattern of treatment failure after surgery is tumor dissemination. A number of prospective clinical trials showed that addition of chemotherapy improves on average the overall survival of patients by approximately 5%. In absolute numbers, this benefit only in Poland translates into 150 additionally saved patients yearly. However, to achieve this gain 3,000 patients need to receive postoperative chemotherapy, whereas only 50% of them will develop disease recurrence.

Currently, the only approved selection criterion for adjuvant treatment is pathologically assessed stage of disease. The prospective randomized studies have shown the benefit from adjuvant chemotherapy in stage II and III NSCLC patients. Still, if these groups are scrutinized, it turns out that around 40% of stage II and 25% of stage III patients will never recur and thus do not need any form of postoperative treatment.

On the other hand, the risk of distant metastases in stage I patients who are currently not administered adjuvant chemotherapy is as high as 30%. Thus, this category of patients apparently includes subgroups of patients with high risk of relapse who might derive benefit from postoperative chemotherapy. However, currently available methods do not allow for identification of these subgroups.

Indeed, the outcome of patients treated surgically is difficult to predict based on clinical and pathological variables, such as stage, histological type, sex or age. Patients with exactly the same characteristics may vary with respect to the risk of metastases and prognosis.

The results of research on molecular markers suggest that transcription assessment may constitute a good prognostic marker in lung cancer. Gene expression profiling (mRNA abundance assessment) was linked to the individual risk of disease dissemination (Bhattacharjee et al. "Classification of human lung carcinomas by mRNA expression profiling reveals distinct adenocarcinoma subclasses". Proc Natl Acad Sci USA 2001; 98: 13790-13795).

It seems that the use of reverse transcription and quantitative polymerase chain reaction (RT-PCR) is the most suited technique for prognostication based on gene expression analysis in tumor tissue.

Endoh et al. in a publication entitled "Prognostic model of pulmonary adenocarcinoma by expression profiling of eight genes as determined by quantitative real-time reverse transcriptase polymerase chain reaction" (J Clin Oncol 2004; 22: 811-819) described a technique enabling quantitation of gene expression in cancer cells. The results of numerous studies based on this technique may ultimately lead to modification of histological classification of lung cancer as well as provide new molecular criteria to refine the staging of this malignancy.

We earlier presented the method of evaluating the risk of distant relapse and prognosis in patients with squamous cell lung cancer using gene expression assessment with RT-PCR (Skrzypski et al. "Three-gene expression signature predicts survival in early-stage squamous cell carcinoma of the lung". Clin Cancer Res 2008; 14: 4794-4799).

Simultaneously to the studies on prognostic role of gene expression (mRNA assessment), in the last few years a new class of molecules: microRNA has attracted attention as a potential prognostic marker.

These molecules are important in many cellular processes, such as embryogenesis, proliferation and differentiation of cells, apoptosis and oncogenesis (reviewed by Earspn et al. "MicroRNAs in development and disease". Clin. Genetics 2008; 74: 296-306).

MicroRNAs are short RNA chains (19-23 nucleotides) that regulate gene expression through inhibiting mRNA translation. This effect is mediated by binding between microRNA and target sequences in mRNA molecules in UTR regions. Given the permissiveness in binding between these molecules, which allows for imperfect complementarity, one microRNA may control the expression of hundreds or thousands of mRNA molecules and thus genes.

The prognostic potential of assessing microRNA expression in primary tumors has been investigated in many cancer types including lung cancer. In a publication by Yu et al. "MicroRNA signature predicts survival and relapse in lung cancer" (Cancer Cell 2008; 13: 48-57), the authors identified a profile consisting of 5 microRNAs, which is highly predictive for prognosis in NSCLC patients in stages I-IIIA.

Currently, a few molecular methods of establishing prognosis in stage I-IIIA NSCLC patients who underwent surgical treatment are known. These methods in principle include: (i) obtaining a sample of primary tumor tissue, (ii) RNA isolation, (iii) reverse transcription of microRNA or mRNA into cDNA, and (iv) establishing the amount of microRNA or mRNA in primary tumor. Finally, the mRNA or microRNA expression value is referred to the reference expression values in a model of disease recurrence prediction, whereby certain expression values are correlated to high and certain to low risk of recurrence.

Herein, we present a method that allows for determination of the risk of distant metastases and prognosis in early (stage I-IIIA) NSCLC. This method is based on assessing the expression in primary tumor tissue of several micro-RNAs (listed below). Particular NSCLCs demonstrate differential expression of the listed microRNAs, which determines different clinical courses of the disease, more specifically a varied propensity to form distant metastases. The method consists of the acquisition of a sample of primary tumor tissue (formalin fixed or frozen) from which RNA is extracted, further retrotranscription of RNA into complementary DNA (cDNA) and its quantification with the use of quantitative PCR method. The microRNA expression value measured in primary tumor tissue is referred to the reference expression values of the microRNA listed in Table 1 (above or below the test cut-off value). These values are correlated to the high and low risk of distant metastases. This in turn enables assignment of patients to the groups with high or low risk of distant recurrence.

Table 1

| Symbol | MicroRNA name | MicroRNA sequence |
| --- | --- | --- |
| A | hsa-miR-10b | UACCCUGUAGAACCGAAUUUGU |
| B | hsa-miR-101* | CAGUUAUCACAGUGCUGAUGCU |
| C | hsa-miR-192* | CUGCCAAUUCCAUAGGUCACAG |
| D | hsa-miR-10a | UACCCUGUAGAUCCGAAUUUGUG |
| E | hsa-miR-874 | CUGCCCUGGCCCGAGGGACCGA |
| F | hsa-miR-10b* | CAGAUUCGAUUCUAGGGGAAUA |
| G | hsa-miR-532-3p | CCUCCCACACCCAAGGCUUGCA |
| H | hsa-miR-622 | ACAGUCUGCUGAGGUUGGAGC |
| I | hsa-miR-508-3p | UGAUUGUAGCCUUUUGGAGUAGA |
| J | hsa-miR-221 | AGCUACAUUGUCUGCUGGGUUU |
| K | hsa-miR-340* | UCCGUCUCAGUUACUUUAUAGC |
| L | hsa-miR-10a* | CAAAUUCGUAUCUAGGGGAAUA |
| M | hsa-miR-222 | AGCUACAUCUGGCUACUGGGU |
| N | hsa-miR-519e | AAGUGCCUCCUUUUAGAGUGUU |
| O | hsa-miR-103 | AGCAGCAUUGUACAGGGCUAUGA |
| P | hsa-miR-149 | UCUGGCUCCGUGUCUUCACUCC |
| R | hsa-miR-532-5p | CAUGCCUUGAGUGUAGGACCGU |
| S | hsa-miR-92a-1* | AGGUUGGGAUCGGUUGCAAUGCU |
| T | hsa-miR-660 | UACCCAUUGCAUAUCGGAGUUG |
| W | hsa-miR-23a | AUCACAUUGCCAGGGAUUUCC |
| Y | hsa-miR-885-5p | UCCAUUACACUACCCUGCCUCU |
| Z | hsa-miR-654-3p | UAUGUCUGCUGACCAUCACCUU |

An exemplary and preferred method of establishing the individual patient prognosis and the risk of NSCLC recurrence relates to a model in which the risk score (RS) is calculated based on the expression levels in primary tumor of at least two microRNAs listed in Table 1.

$$RS = micro\ RNA\ A + micro\ RNA\ B + ... + micro\ RNA\ Z$$

micro RNA A...Z - partial risk value

wherein the RS value is calculated by adding the "partial risk values" incurred from the expressions of the microRNA that make up a given risk score. Partial risk values are expressed as numerical values and are ascribed according to the recurrence prediction model in which an expression above or below the test cut-off value for a given microRNA results in either high (preferably "1") or low (preferably "0") partial risk value.

The embodiment of the abovementioned method comprises the entire kit necessary for performing the assessment of microRNA expression. Such kit contains at least: (i) reagents to carry out reverse transcription of the microRNA molecules into cDNA (specific primers, reverse transcriptase, buffers and other reagents); (ii) one primer set to carry out quantitative PCR reaction (at least one primer hybridizes with at least a fragment of the cDNA sequence corresponding to microRNA molecule from the list described in claim 1, polymerase, buffers and other reagents).

The application of the invention will allow for assessment of the individual risk of recurrence in NSCLC patients and will provide a precise tool for selecting particular patients to adjuvant therapies based on their individual risk. Only patients with high risk of recurrence would be administered adjuvant chemotherapy, whereas low risk patients might be spared additional treatment. As a final result, improved treatment outcomes, as well as reduction in toxicity and treatment costs, may be expected.

The invention is further explained by the following examples:

Example 1

Total RNA containing microRNA was isolated from tumor tissue with miRNeasy Mini Kit (50) (Qiagen) 217004. The concentration and quality of RNA was assessed with RNA Lab Chip (Bianalyzer Agilent 2100). Subsequently, RNA was retrotranscribed to cDNA (RT reaction) with TaqMan MicroRNA RT kit 4366596 - Applied Biosystems with the use of specific stem-loop primers specific to microRNAs (MegaPlex RT Cat. no. 4401091 Applied Biosystems) in accordance with the manufacturers' recommendations.

MicroRNA (cDNA resultant from RT reaction) was quantitied with quantitative PCR reaction using specific primers pairs and fluorescent TaqMan probes and polymerase with 5' nuclease activity in microfluidic cards (TaqMan Low Density Arrays - Part Number 4400238 Applied Biosystems) in HT 7900 cycler (Applied Biosystems) reaction conditions in accordance with manufacturers' recommendations (Applied Biosystems).

Raw expression results (Ct values) were obtained through SDS.2.1 (Applied Biosystems) software and the expression was normalized against the expression of U6 RNA.

The values of microRNA expression are correlated with the reference expression in a model of risk prediction (distant metastases prediction). In this method, certain values correspond to high risk of recurrence, whereas others to low risk of recurrence. The expression corresponding to these reference values denotes high or low risk of disease recurrence in the individual patient.

Table 2

| Symbol | MicroRNA name | MicroRNA sequence | Statistical significance level (p value) |
|---|---|---|---|
| A | hsa-miR-10b | UACCCUGUAGAACCGAAUUUGU | 0,0002 |
| B | hsa-miR-101* | CAGUUAUCACAGUGCUGAUGCU | 0,005 |
| C | hsa-miR-192* | CUGCCAAUUCCAUAGGUCACAG | 0,011 |
| D | hsa-miR-10a | UACCCUGUAGAUCCGAAUUUGUG | 0,025 |
| E | hsa-miR-874 | CUGCCCUGGCCCGAGGGACCGA | 0,021 |
| F | hsa-miR-10b* | CAGAUUCGAUUCUAGGGGAAUA | 0,017 |
| G | hsa-miR-532-3p | CCUCCCACACCCAAGGCUUGCA | 0,031 |
| H | hsa-miR-622 | ACAGUCUGCUGAGGUUGGAGC | 0,025 |
| I | hsa-miR-508-3p | UGAUUGUAGCCUUUUGGAGUAGA | 0,026 |

(continued)

| Symbol | MicroRNA name | MicroRNA sequence | Statistical significance level (p value) |
|---|---|---|---|
| J | hsa-miR-221 | AGCUACAUUGUCUGCUGGGUUU | 0,027 |
| K | hsa-miR-340* | UCCGUCUCAGUUACUUUAUAGC | 0,028 |
| L | hsa-miR-10a* | CAAAUUCGUAUCUAGGGGAAUA | 0,029 |
| M | hsa-miR-222 | AGCUACAUCUGGCUACUGGGU | 0.05 |
| N | hsa-miR-519e | AAGUGCCUCCUUUUAGAGUGUU | 0,045 |
| O | hsa-miR-103 | AGCAGCAUUGUACAGGGCUAUGA | 0,032 |
| P | hsa-miR-149 | UCUGGCUCCGUGUCUUCACUCC | 0,033 |
| R | hsa-miR-532-5p | CAUGCCUUGAGUGUAGGACCGU | 0,038 |
| S | hsa-miR-92a-1* | AGGUUGGGAUCGGUUGCAAUGCU | 0,041 |
| T | hsa-miR-660 | UACCCAUUGCAUAUCGGAGUUG | 0,042 |
| W | hsa-miR-23a | AUCACAUUGCCAGGGAUUUCC | 0,042 |
| Y | hsa-miR-885-5p | UCCAUUACACUACCCUGCCUCU | 0.05 |
| Z | hsa-miR-654-3p | UAUGUCUGCUGACCAUCACCUU | 0,048 |

For example, underneath are the metastases free survival (MFS) probability curves according to high vs. low expression of microRNA 221 (AGCUACAUUGUCUGCUGGGUUU).

Metastases free survival vs MicroRNA 221 expression
(40th percentile expression value as cutt-off)

The difference between the MFS in the high and low risk groups (using the 40th percentile of 221 expression in the entire group of patients as a cut-off value) was highly significant (p=0.011). The low expression of microRNA 221 was related to the high risk of distant recurrence after surgical treatment (almost 60% at 5 years).

Another example presents MFS probability curves according to high vs. low expression of microRNA 10b

(UACCCUGUAGAACCGAAUUUGU).

Metastases free survival vs. MicroRNA 10b expression
(40th percentile expression value as cut-off)

The difference between the median MFS in the high and low risk groups (using the 40th percentile of 10b expression in the entire group of patients as a cut-off value) was highly significant (p=0.001). The low expression of microRNA 10b was related to the high risk of distant recurrence after surgical treatment (almost 70% at 5 years).

Another example presents MFS probability curves according to high vs. low expression of microRNA of micro-RNA 192* CUGCCAAUUCCAUAGGUCACAG.

Metastases free survival vs MicroRNA 192 expression (60th percentile expression value as cutt-off)

The difference between the median MFS in the high and low risk groups (using the 40th percentile of 10b expression in the entire group of patients as a cut-off value) was highly significant (p=0.03). The high expression of microRNA 192* was related to the high risk of distant recurrence after surgical treatment (almost 60% at 5 years).

All microRNAs that are listed in this patent application are significantly related to the MFS - the corresponding p-values are listed in Table 2. High expression (i.e. higher than median in the entire group) of the following microRNAs is related to the high risk of NSCLC recurrence after surgical treatment: 92a,192* and 622

Low expression of the following microRNAs (i.e. lower than median in the entire group) is related to the high risk of NSCLC recurrence after surgical treatment: 10b, 10b*, 10a, 23a, 101, 103, 149, 221, 222, 340, 508, 519e, 532_3p, 532_5p, 654, 660, 874 and 885-5p.

The cut-offs set at the level of the median expression value or the 40th percentile expression value for a given microRNA are just exemplary and should by no means be viewed as limiting the claim in this invention. The final cut-off values will be determined after performing the expression analysis of the above-mentioned microRNAs in a larger series of patients.

Example 2

The method is the same as in example 1, wherein at least 2 microRNAs or more are chosen from the list of the 22 microRNAs to make up a risk index that reflects the risk of recurrence of constituting microRNAs:

hsa-miR-532-5p
hsa-miR-92a-1*
hsa-miR-192*
hsa-miR-10b

Risk index in this example takes the following form:

$$RS = microRNA\ 532\text{-}5p + microRNA\ 92a + microRNA192* + microRNA\ 10b$$

If the expression of the microRNA is indicative of the high risk according to the recurrence risk model, the "partial risk" for a given microRNA takes the value of "1"; if the expression of a given microRNA was indicative of the low risk according to the recurrence risk model the "partial risk" takes the value of "0".

The risk score was calculated for each patient. Next, the MFS curves were generated for the groups of patients with RS values above and below the cut-off value for RS (the 60th percentile of the RS value in the entire group).

The probability of MFS was compared between the two groups.

Metastases free survival vs risk score values
(60th percentile expression value as cutt-off)

Example 3

The method is the same as in example 1, wherein at least 2 microRNAs or more are chosen from the list of the 22 microRNAs to make up a risk index that reflects the risk of recurrence of constituting microRNAs:

    hsa-miR-101*-4395254
    hsa-miR-532-5p-4380928
    hsa-miR-222-4395387
    hsa-miR-192*-4395383
    hsa-miR-10b-4395329

In this invention embodiment, the "partial risk" values are additionally weighted by a weighting factor (in this instance, these factors were derived from multivariate MFS analysis).

$$RS = (-2,7*MiR101)+(-0,6*MiR222)+(-1,2*MiR532\text{-}3p)+(0,78*MiR192)+(-2*MiR10b)$$

Next, the MFS curves were generated for the groups of patients with RS values above and below the cut-off value for RS (the 60th percentile of the RS value in the entire group).

The probability of MFS was compared between the two groups.

Metastases free survival vs risk score values
(60th percentile expression value as cutt-off)

Example 4.

The method analogous to the examples 1-3 wherein to carry out the necessary steps of the method a proprietary kit (set of reagents) is provided. The kit contains all necessary reagents to isolate microRNA from tumor tissue and to perform reverse transcription and quantitative polymerase chain reaction. Among other things, the kit contains specifically primers, of which at least one hybridizes with at least a part of the listed microRNAs that enable reverse transcriptions reaction and the reagents for quantitative polymers chain reaction: primers that hybridize with at least a part of the cDNA sequences that correspond to microRNA molecules, enzymes and other reagents necessary for quantitative polymers chain reaction.

**Claims**

1. A method of determination of the risk of distant metastases in surgically treated patients with non-small cell lung cancer in stage I-IIIA wherein microRNA is extracted from a sample of primary tumor tissue, retrotranscribed into complementary DNA by reverse transcription, quantity of microRNA hsa-miR-660 is examined with the use of quantitative PCR method and the expression value of this microRNA is referred to the corresponding microRNA reference expression values in a disease recurrence prediction model in which low expression value of microRNA hsa-miR-660 is correlated to the high risk of distant metastases.

2. A method of claim 1, wherein quantity of at least one further microRNA from Table 1 is examined with the use of quantitative PCR method and the expression value of each further microRNA is referred to the corresponding microRNA reference expression values in a disease recurrence prediction model in which certain expression values are correlated to the high or low risk of distant metastases as in Table 1.

Table 1

| | MicroRNA name | MicroRNA sequence | MARKER Expression level of microRNA indicating high risk of distant metastases |
|---|---|---|---|
| 1 | hsa-miR-10b | UACCCUGUAGAACCGAAUUUGU | LOW |

(continued)

|  | MicroRNA name | MicroRNA sequence | MARKER Expression level of microRNA indicating high risk of distant metastases |
|---|---|---|---|
| 2 | hsa-miR-101* | CAGUUAUCACAGUGCUGAUGCU | LOW |
| 3 | hsa-miR-192* | CUGCCAAUUCCAUAGGUCACAG | HIGH |
| 4 | hsa-miR-10a | UACCCUGUAGAUCCGAAUUUGUG | LOW |
| 5 | hsa-miR-874 | CUGCCCUGGCCCGAGGGACCGA | LOW |
| 6 | hsa-miR-10b* | CAGAUUCGAUUCUAGGGGAAUA | LOW |
| 7 | hsa-miR-532-3p | CCUCCCACACCCAAGGCUUGCA | LOW |
| 8 | hsa-miR-622 | ACAGUCUGCUGAGGUUGGAGC | HIGH |
| 9 | hsa-miR-508-3p | UGAUUGUAGCCUUUUGGAGUAGA | LOW |
| 10 | hsa-miR-221 | AGCUACAUUGUCUGCUGGGUUU | LOW |
| 11 | hsa-miR-340* | UCCGUCUCAGUUACUUUAUAGC | LOW |
| 12 | hsa-miR-10a* | CAAAUUCGUAUCUAGGGGAAUA | LOW |
| 13 | hsa-miR-222 | AGCUACAUCUGGCUACUGGGU | LOW |
| 14 | hsa-miR-519e | AAGUGCCUCCUUUUAGAGUGUU | LOW |
| 15 | hsa-miR-103 | AGCAGCAUUGUACAGGGCUAUGA | LOW |
| 16 | hsa-miR-149 | UCUGGCUCCGUGUCUUCACUCC | LOW |
| 17 | hsa-miR-532-5p | CAUGCCUUGAGUGUAGGACCGU | LOW |
| 18 | hsa-miR-92a-1* | AGGUUGGGAUCGGUUGCAAUGCU | HIGH |
| 19 | hsa-miR-23a | AUCACAUUGCCAGGGAUUUCC | LOW |

3. A method of claim 2, wherein the risk of distant metastases is established by means of the risk scores (RS), which are calculated based on the expression levels in primary tumor of each examined microRNAs, wherein:

$$RS = microRNA\ 1 + microRNA\ 2 + ... + microRNA\ X,$$

where the expression 'microRNA N' (e.g. microRNA hsa-miR-660, etc.) - denotes *partial risk value,*
and wherein the RS value is calculated by adding the *partial risk values* incurred from the expression of each examined microRNA constituting a given risk score,
and wherein *partial risk values* are obtained according to the recurrence prediction model, in which a high risk value is expressed as numerical value,
preferably "1" and a low risk value is expressed as numerical value, preferably "0".

4. A method of any of claims from 1 to 3, wherein the whole kit which comprises set of primers of which at least one hybridizes with a fragment of the examined microRNA, which enable carrying out reverse transcription and quantitative PCR reaction, and preferably comprises such reagents as buffers, enzymes and possibly other reagents for carrying out reverse transcription and quantitative PCR reaction is used.

**Patentansprüche**

1. Verfahren zum Bestimmen des Risikos von Fernmetastasen bei chirurgisch behandelten Patienten mit nicht-kleinzelligem Lungenkrebs im Stadium I-IIIA, wobei aus einer Probe von Primärtumorgewebe MikroRNA isoliert und durch reverse Transkription zurück in komplementäre DNA transkribiert wird, die Menge an MikroRNA-hsa-miR-660 durch Anwendung des quantitativen PCR-Verfahrens untersucht wird, und der Expressionswert dieser MikroRNA mit den entsprechenden MikroRNA-Referenzexpressionswerten in einem Modell zur Vorhersage von Krankheitsrezidiven, in dem ein niedriger Expressionswert von MikroRNA-hsa-miR-660 mit dem hohen Risiko von Fernmetastasen korreliert, in Bezug gesetzt wird.

2. Verfahren nach Anspruch 1, wobei die Menge von mindestens einer weiteren MikroRNA aus Tabelle 1 durch Anwendung des quantitativen PCR-Verfahrens untersucht wird, und der Expressionswert jeder weiteren MikroRNA mit den entsprechenden MikroRNA-Referenzexpressionswerten in einem Modell zur Vorhersage von Krankheitsrezidiven, in dem wie in Tabelle 1 angegeben bestimmte Expressionswerte mit dem hohen oder niedrigen Risiko von Fernmetastasen korrelieren, in Bezug gesetzt wird.

Tabelle 1

| | Bezeichnung der MikroRNA | MikroRNA-Sequenz | MARKER Expressionsniveau von MikroRNA, das auf ein hohes Risiko von Fernmetastasen hinweist |
|---|---|---|---|
| 1 | hsa-miR-10b | UACCCUGUAGAACCGAAUUUGU | NIEDRIG |
| 2 | hsa-miR-101* | CAGUUAUCACAGUGCUGAUGCU | NIEDRIG |
| 3 | hsa-miR-192* | CUGCCAAUUCCAUAGGUCACAG | HOCH |
| 4 | hsa-miR-10a | UACCCUGUAGAUCCGAAUUUGUG | NIEDRIG |
| 5 | hsa-miR-874 | CUGCCCUGGCCCGAGGGACCGA | NIEDRIG |
| 6 | hsa-miR-10b* | CAGAUUCGAUUCUAGGGGAAUA | NIEDRIG |
| 7 | hsa-miR-532-3p | CCUCCCACACCCAAGGCUUGCA | NIEDRIG |
| 8 | hsa-miR-622 | ACAGUCUGCUGAGGUUGGAGC | HOCH |
| 9 | hsa-miR-508-3p | UGAUUGUAGCCUUUUGGAGUAGA | NIEDRIG |
| 10 | hsa-miR-221 | AGCUACAUUGUCUGCUGGGUUU | NIEDRIG |
| 11 | hsa-miR-340* | UCCGUCUCAGUUACUUUAUAGC | NIEDRIG |
| 12 | hsa-miR-10a* | CAAAUUCGUAUCUAGGGGAAUA | NIEDRIG |
| 13 | hsa-miR-222 | AGCUACAUCUGGCUACUGGGU | NIEDRIG |
| 14 | hsa-miR-519e | AAGUGCCUCCUUUUAGAGUGUU | NIEDRIG |
| 15 | hsa-miR-103 | AGCAGCAUUGUACAGGGCUAUGA | NIEDRIG |
| 16 | hsa-miR-149 | UCUGGCUCCGUGUCUUCACUCC | NIEDRIG |
| 17 | hsa-miR-532-5p | CAUGCCUUGAGUGUAGGACCGU | NIEDRIG |
| 18 | hsa-miR-92a-1* | AGGUUGGGAUCGGUUGCAAUGCU | HOCH |
| 19 | hsa-miR-23a | AUCACAUUGCCAGGGAUUUCC | NIEDRIG |

3. Verfahren nach Anspruch 2, wobei das Risiko von Fernmetastasen mithilfe der Risiko-Scores (RS) festgestellt wird, welche auf der Grundlage der Expressionsniveaus jeder untersuchten MikroRNA im Primärtumor berechnet werden, wobei gilt:

$$RS = MikroRNA\ 1 + MikroRNA\ 2 + ... + MikroRNA\ X,$$

wobei der Ausdruck "MikroRNA-N" (z. B. MikroRNA-hsa-miR-660 usw.) - einen *partiellen Risikowert* bezeichnet, und wobei der RS-Wert berechnet wird, indem die *partiellen Risikowerte,* die durch die Expression von jeder untersuchten MikroRNA anfallen und einen bestimmten Risiko-Score darstellen, addiert werden und wobei *partielle*

*Risikowerte* nach dem Modell zur Vorhersage von Rezidiven erhalten werden, In dem ein hoher Risikowert als numerischer Wert, vorzugsweise "1", ausgedrückt wird und ein niedriger Wert als numerischer Wert, vorzugsweise "0", ausgedrückt wird.

4. Verfahren nach einem der Ansprüche 1 bis 3, wobei das gesamte Kit verwendet wird, welches Sätze von Primern, von denen mindestens einer an ein Fragment der untersuchten MikroRNA hybridisiert, welches es ermöglicht, eine reverse Transkription und eine quantitative PCR-Reaktion durchzuführen, und vorzugsweise Reagenzien wie Puffer, Enzyme und möglicherweise andere Reagenzen zum Durchführen einer reversen Transkription und einer quantitativen PCR-Reaktion umfasst.

**Revendications**

1. Procédé de détermination du risque de métastases distantes chez des patients traités chirurgicalement et ayant un cancer du poumon non à petites cellules de stade I à IIIA, dans lequel un microARN est extrait d'un échantillon de tissu tumoral primaire, rétrotranscrit en ADN complémentaire par transcription inverse, une certaine quantité de microARN hsa-miR-660 est examinée en utilisant comme méthode la PCR quantitative et la valeur d'expression de ce microARN se réfère aux valeurs d'expression de référence du microARN correspondantes dans un modèle prédictif de récidive de la maladie dans lequel une valeur d'expression faible du microARN hsa-miR-660 est corrélée au risque élevé de métastases distantes.

2. Procédé selon la revendication 1, dans lequel une certaine quantité d'au moins un autre microARN du tableau 1 est examinée en utilisant comme méthode la PCR quantitative et la valeur d'expression de chaque autre microARN se réfère aux valeurs d'expression de référence du microARN correspondantes dans un modèle prédictif de récidive de la maladie dans lequel certaines valeurs d'expression sont corrélées avec le risque élevé ou faible de métastases distantes comme l'indique le tableau 1.

Tableau 1

| | Nom du microARN | Séquence du microARN | MARQUEUR Niveau d'expression du microARN indiquant un risque élevé de métastases distantes |
|---|---|---|---|
| 1 | hsa-miR-10b | UACCCUGUAGAACCGAAUUUGU | FAIBLE |
| 2 | hsa-miR-101* | CAGUUAUCACAGUGCUGAUGCU | FAIBLE |
| 3 | hsa-miR-192* | CUGCCAAUUCCAUAGGUCACAG | ÉLEVÉ |
| 4 | hsa-miR-10a | UACCCUGUAGAUCCGAAUUUGUG | FAIBLE |
| 5 | hsa-miR-874 | CUGCCCUGGCCCGAGGGACCGA | FAIBLE |
| 6 | hsa-miR-10b* | CAGAUUCGAUUCUAGGGGAAUA | FAIBLE |
| 7 | hsa-miR-532-3p | CCUCCCACACCCAAGGCUUGCA | FAIBLE |
| 8 | hsa-miR-622 | ACAGUCUGCUGAGGUUGGAGC | ÉLEVÉ |
| 9 | hsa-miR-508-3p | UGAUUGUAGCCUUUUGGAGUAGA | FAIBLE |
| 10 | hsa-miR-221 | AGCUACAUUGUCUGCUGGGUUU | FAIBLE |
| 11 | hsa-miR-340* | UCCGUCUCAGUUACUUUAUAGC | FAIBLE |
| 12 | hsa-miR-10a* | CAAAUUCGUAUCUAGGGGAAUA | FAIBLE |

(suite)

| | Nom du microARN | Séquence du microARN | MARQUEUR Niveau d'expression du microARN indiquant un risque élevé de métastases distantes |
|---|---|---|---|
| 13 | hsa-miR-222 | AGCUACAUCUGGCUACUGGGU | FAIBLE |
| 14 | hsa-miR-519e | AAGUGCCUCCUUUUAGAGUGUU | FAIBLE |
| 15 | hsa-miR-103 | AGCAGCAUUGUACAGGGCUAUGA | FAIBLE |
| 16 | hsa-miR-149 | UCUGGCUCCGUGUCUUCACUCC | FAIBLE |
| 17 | hsa-miR-532-5p | CAUGCCUUGAGUGUAGGACCGU | FAIBLE |
| 18 | hsa-miR-92a-1* | AGGUUGGGAUCGGUUGCAAUGCU | ÉLEVÉ |
| 19 | hsa-miR-23a | AUCACAUUGCCAGGGAUUUCC | FAIBLE |

**3.** Procédé selon la revendication 2, dans lequel le risque de métastases distantes est établi au moyen des scores de risque (SR), qui sont calculés d'après les niveaux d'expression dans la tumeur primaire de chaque microARN examiné, où :

$$SR = microARN\ 1 + microARN\ 2 + ... + microARN\ X,$$

l'expression « microARN N » (par exemple, microARN hsa-miR-660, etc.) désignant une *valeur de risque partiel* et la valeur SR étant calculée en additionnant les *valeurs de risque partiel* découlant de l'expression de chaque microARN examiné constituant un score de risque donné et les *valeurs de risque partiel* étant obtenues selon le modèle prédictif de récidive dans lequel une valeur de risque élevé est exprimée sous forme de valeur numérique, de préférence « 1 » et une valeur de risque faible est exprimée sous forme de valeur numérique, de préférence « 0 ».

**4.** Procédé selon l'une quelconque des revendications 1 à 3, dans lequel le kit complet, comprenant un jeu d'amorces dont l'une d'elles au moins s'hybride avec un fragment du microARN examiné, permettant de réaliser une transcription inverse et une PCR quantitative et comprenant de préférence des réactifs tels que des tampons, des enzymes et éventuellement d'autres réactifs pour effectuer une transcription inverse et une PCR quantitative, est utilisé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BHATTACHARJEE et al.** Classification of human lung carcinomas by mRNA expression profiling reveals distinct adenocarcinoma subclasses. *Proc Natl Acad Sci USA,* 2001, vol. 98, 13790-13795
- **ENDOH et al.** Prognostic model of pulmonary adenocarcinoma by expression profiling of eight genes as determined by quantitative real-time reverse transcriptase polymerase chain reaction. *J Clin Oncol,* 2004, vol. 22, 811-819
- **SKRZYPSKI et al.** Three-gene expression signature predicts survival in early-stage squamous cell carcinoma of the lung. *Clin Cancer Res,* 2008, vol. 14, 4794-4799
- **EARSPN et al.** MicroRNAs in development and disease. *Clin. Genetics,* 2008, vol. 74, 296-306
- **YU et al.** MicroRNA signature predicts survival and relapse in lung cancer. *Cancer Cell,* 2008, vol. 13, 48-57